# EUROPEAN PATENT APPLICATION

(11) **EP 1 564 305 A2**
(43) Date of publication of application: **17.08.2005**
(21) Application number: 04290378.1
(22) Date of filing: 12.02.2004
(51) Int. Cl.: C12Q 1/68, G01N 33/574

(54) **Means for detecting and treating cancer cells resistant to therapeutic agents**

(71) Applicant: Institut Curie, 75248 Paris Cedex 05 (FR); CENTRE NATIONAL DE LA RECHERCHE SCIENTIFIQUE (CNRS), 75794 Paris Cedex 16 (FR)
(72) Inventor: Poupon, Marie-France, 94260 Fresnes (FR); Judde, Jean-Gabriel, 94110 Arcueil (FR); Bras-Goncalves, Rui, 34090 Montpellier (FR)
(74) Representative: Peaucelle, Chantal

(57) **Abstract**

The invention relates to tumor markers predictive of the tumor cells resistance to inhibitors of RTK activity consisting of phosphatase/kinase expression ratio as measured in a tumor sample.

## Description

The invention relates to means for detecting and treating cancer cells resistant to therapeutic agents, more particularly to receptor tyrosine kinase (RTK in short) inhibitors.

Members of the RTK family play an important role in the proliferation of cancer cells. Aberrant signaling through the epidermal growth factor receptor (EGFR), a member of the ErbB family of RTKs, is associated with neoplastic cell proliferation, migration, invasion, resistance to apoptosis and angiogenesis.

Upon ligand binding, the EGFR dimerizes, and undergoes autophosphorylation at specific tyrosine residues of the intracellular domain. The phosphorylated tyrosines then serve as docking sites for proteins which, in turn, activate downstream signaling pathways, including the Ras/MEK/Erk and the PI3K/Akt pathway, which regulate transcription factors and other proteins involved in the above-mentioned biological responses.

Beside cell proliferation, the balance between cell survival and cell death is a determinant factor in the growth of tumors and their response to therapeutic agents. It is known that cell proliferation and apoptosis are controlled by highly regulated mitogen-activated protein (MAP) kinases, including ERK (extracellular signal-regulated kinase), JNK (c-JUN NH₂-terminal protein kinase), and p38.

MAP kinases participate in signal transduction pathways through which cells respond functionally to external messages or to extracellular stresses. Different cell stimuli preferentially activate distinct MAP kinases. Hence, growth factors (such as EGF) and oncogenes are linked to activation of ERK, whereas inflammatory cytokines, growth factor deprivation and a number of cell stresses lead preferentially to activation of JNK and p38, which are also called stress-activated protein kinases (SAPK). The different actors involved in cell death, survival and proliferation constitute a network that is highly regulated in normal cells, and variably altered in cancer cells.

Experimental studies suggest that ERK MAP kinases are involved in cell cycle progression and mitogenesis, oncogenic transformation and metastasis, differenciation and survival, whereas JNK and p38 participate in signaling pathways leading to apoptosis. How tumor cells regulate the decision point between either proliferation or survival and cell death is context-dependent and results from a complex relationship between the Akt and Erk survival pathways and the SAPK death pathways.

In particular, several studies indicated that concomitant inhibition of the ERK or PI3K/Akt signaling pathways with activation of the SAPK pathways can switch the equilibrium toward apoptosis in various experimental tumor systems.

Temporal activation of MAP kinases is regulated positively by upstream kinases and negatively by several classes of phosphatases, including the serine/threonine phosphatase PP2A, over 50 tyrosine-specific phosphatases (PTPs) and a growing family of a subclass of PTPs that possess activity for dephosphorylating both phosphotyrosine and phosphothreonine residues, termed dual specificity phosphatases (DSPs).

Tight control of DSP gene induction, combined with their differential binding and catalytic activation by a specific repertoire of MAP kinases, provides a sophisticated mechanism for rapid and targeted inactivation of selected MAP kinase activities. Hence, the DSPs MKP-3 and M3/6 are highly specific for inactivating ERK1/2 and either JNK or p38 respectively, while CL100/MKP-1 and MKP-5 act preferentially on JNK and p38.

Several recent studies suggest that MKP-1 is involved in the progression of human cancer by protecting tumor cells against apoptosis. First, MKP-1 expression is induced by low oxygen conditions found in solid tumor microenvironments, which may represent a mechanism to protect tumor cells from apoptosis induced by the activation of JNK in response to hypoxic stress. Second, MKP-1 expression is increased in several tumor types, including breast, prostate and ovarian carcinomas, and was found associated with cell survival in prostate tumor samples obtained from patients treated by androgen deprivation.

The high frequency of abnormalities in RTK signaling, particularly EGFR, in human tumors and laboratory studies showing that inhibition of EGFR can impair tumor growth, means that EGFR is an attractive target for the development of cancer therapeutics.

High expression of the EGFR and/or its ligands is common in several tumor types, including non-small cell lung carcinoma (NSCLC), gliomas, head and neck cancer (HNSCC), breast cancer, and ovarian cancer, and correlates with more aggressive disease and resistance to chemotherapy.

Several inhibitors of the EGFR and other RTKs are in development. Gefitinib (ZD1839, Iressa) is an orally administered, selective and reversible inhibitor of EGFR that competitively inhibits the binding of ATP required for receptor autophosphorylation and kinase activation. Preclinical studies suggest that gefitinib and other EGFR-targeting agents inhibit tumor cell proliferation and angiogenesis, induce apoptosis, and show additive or synergistic cytotoxic effects on tumor cells when used in combination with standard cancer therapies.

NSCLC is the most frequent cancer in the world and one of the most lethal as well, with one million deaths worldwide in 2000. Standard chemotherapy for advanced and metastatic NSCLC is based on various cytotoxic drug combinations. Although chemotherapy results in a modest survival benefit, four different reference chemotherapeutic combinations show similar therapeutic effectiveness, meaning that a plateau has been reached with conventional chemotherapy. Moreover, relapses following first line chemotherapy are constant and treatment is associated with severe toxicity. New therapeutic approaches with improved efficacy and better tolerability are therefore urgently needed for NSCLC and other chemoresistant tumors.

In May, 2003, the US Food and Drug Administration approved gefitinib for the treatment of patients with advanced NSCLC previously treated with chemotherapy. This approval was based on results of a phase II clinical study in which 142 patients with refractory disease showed a 10% response rate to gefitinib. The approval of the drug was granted despite negative results from two randomized controlled trials in over 2000 previously untreated NSCLC patients, which showed no benefit in survival, tumor response, or time to progression when gefitinib was added to chemotherapy. Among other possible reasons, the failure to demonstrate a benefit might have been an important dilution of the benefit in a small cohort of patients with tumors sensitive to EGFR inhibition by a larger cohort of patients with insensitive tumors.

Preclinical studies are therefore needed to identify and validate predictive factors to select patients with disease likely to respond to EGFR inhibitors. Findings from phase II clinical studies usually do not show a correlation between the degree of EGFR expression and response to therapy.

The inventors have looked for genes that could be involved in the response of human tumors to the growth inhibitory effect of a competitive and reversible inhibitor of EGFR.

Growth-inhibition studies conducted *in vivo* with several human tumor xenografts and *in vitro* with human HNSCC cell lines, identified tumors responsive and tumors refractory to said EGFR inhibitor. Genes whose expression levels were highly correlated with response to said inhibitor were identified. Expression ratio thereof was associated with resistance to said inhibitor.

In support of this finding, the inventors have found that a treatment combining RTK inhibitor activity and an inhibitor of MKP-1 activity has a synergistic anti-tumor effect on RTK inhibitor -resistant tumors.

An object of the invention is then to provide markers and methods for predicting tumor response to RTK activity inhibition.

Another object of the invention is to provide means for potentiating the anti-tumor effect of RTK activity inhibitors.

The invention thus relates to tumor markers predictive of the tumor cells resistance to inhibitors of RTK activity consisting of phosphatase/kinase expression ratio as measured in a tumor sample.

Such an expression level ratio appears indeed to be highly correlated with the response to inhitors of RTK. A high expression ratio was shown to be associated with resistance to RTK inhibitor.

The invention particularly relates to markers consisting of MKP-1/MKK7 mRNA ratio.

As shown in the examples, a high amount of MKP-1 (which codes for a DSP which inactivates JNK) relative to MKK7 (which codes for a kinase that is a positive upstream regulator of JNK) is indicative of effective inhibition of JNK activity by MKP-1. Such a ratio constitutes a positive index of JNK signaling inhibition.

In other embodiments of the invention, the following mRNA levels ratios may be used to assess the activity level of the corresponding MAP kinases (sequences correspond to GenBank accession numbers):
For the activation level of JNK1/2/3 (seq. NM 002750, NM 139046, NM 139047, NM 139049, NM 002752, NM 139068, NM 139069, NM 139070, NM 002753, NM 138980, NM 138981, NM 138982), the mRNA ratios of hVH2 (seq. NM 001394, NM 057158) and/or MKP7 (seq. NM 030640) and/or MKP5 (seq. NM 007207, NM 144728, NM 144729) and/or hVH1 (seq. NM 004417) and/or hVH5 (seq. NM 004420) to MKK7 (seq. NM 145185) and/or MKK4 (seq. NM 003010) and/or CDC 42 (seq. NM 001791, NM 044472) and/or MAP4K3 (seq. NM 003618).
For the activation level of MAPK11 (seq. NM 002751, NM 138993) and of MAPK14 (seq. NM 001315, NM 139012, NM 139013, NM 139014) and of MAPK12 (seq; NM 002969), the mRNA ratio of hVH2 (seq. NM 001394, NM 057158) and/or MKP7 (seq. NM 030640) and/or MKP5 (seq. NM 007207, NM 144728, NM 144729) and/or hVH1 (seq. NM 004417) and/or hVH5 (seq. NM 004420) to MKK6 (seq. NM 002758, NM 031988) and/or MKK4 (seq. NM 003010).

The invention also relates to a method for predicting tumor response to RTK inhibition, comprising measuring phosphatase/kinase mRNA level in tumors.

The inventors have found that the degree of activation of downstream kinase and the sensitivity of malignant tumors to inhibition of RTK activity can be predicted with the mRNA level of said genes involved in said signaling pathways.

In a preferred embodiment, said method comprises the measurement of MKP-1/MKK7 mRNA ratio.

In other embodiments, said method comprises the measurement of mRNA levels ratios such as above defined to assess the activity level of the specific MAP kinases.

Advantageously, said expression level is measured by using a real-time quantitative assay and calculating the relative mRNA levels by the comparative Ct method following validation by titration curve analysis (according to Applied Biosystems' recommendations)

Oligonucleotides primers specific for the MKP-1 and MKK7 cDNA sequences are for example:
For MKP-1: Forward sequence SEQ ID N°1: 5′-GACTTCATAGACTCCATCAAGAATGC Reverse sequence SEQ ID N°2: 5'-GGCAGTGGACAAACACCCTT
For MKK7

Forward sequence SEQ ID N°3: 5'-GGACCTGGATGTGGTGCTG

Reverse sequence SEQ ID N°4: GAAGACGTCCGTGTTGGTGA

The invention also relates to the use of said tumor markers to select patients with tumors susceptible to respond to RTK inhibitors.

Advantageously, such a use further comprises administering to a patient an effective amount of a RTK inhibitor in combination with an inhibitor of phosphatase activity.

The treatment combining downstream MAP kinase inactivation, via RTK inhibition, with phosphatase inactivation resulting either spontaneously from low constitutive phosphatase expression, in case of RTK-inhibitor sensitive tumor, or from inhibition of phosphatase in cells with a high phosphatase/kinase mRNA ratio, in the case of RTK inhibitor-resistant tumors treated with an inhibitor of phosphatase, inhibits tumor growth in part through induction of apoptosis. The results given in the examples show a synergistic induction of apoptosis in cells treated by a combination of RTK-inhibitor and a phosphatase-inhibitor.

The invention thus relates to a method of treatment combining inhibition of a phosphatase activity with suppression of MAP kinase. Such a method of treatment results in a synergistic anti-tumor effect in tumors with a high phosphatase/kinase mRNA ratio, and have a significant anti-tumor activity in tumors that are resistant to either treatment alone.

Advantageously, such a combined treatment also allows a reduction in the effective dose of RTK inhibitor, whose toxicity often remains limiting in patients.

Such a method is particularly useful for treating cancers in which RTK signaling plays an important role for cell proliferation.

The invention is then also directed to pharmaceutical compositions comprising an effective amount of a RTK inhibitor in combination with an inhibitor of phosphatase activity.

In a preferred embodiment, the RTK inhitor is gefitinib. In another preferred embodiment, the inhibitor of phosphatase is an isothiocyonate, such as PEITC. In a more preferred embodiment, the treatment comprises using in combination, together or sequencially, gefitinib and PEITC.

The doses will be advantageously of 50-100% the MTD (Maximal Tolerated Dose) for each agent. Each agent will administred at repeated doses, for example alternatively, each day, during.5-10 days.

Other characteristics and advantages of the invention will be disclosed hereinafter, with reference to the figures 1 to 5, which respectively represent:
Figure 1: Result of experiments with radiolabelled cDNAs from IC9 and SC131 NSCLC xenografts hybridized to MAP kinase Superarray membranes, showing high expression of MKP-1 mRNA in SC131 and its absence in IC9.
Figure 2: Histogram showing the distribution of MKP-1/MKK7 mRNA level ratios in 4 human NSCLC xenografts and in 3 human HNSCC cell lines.
Figure 3-a:Effect of gefitinib and PEITC alone or combined on the growth and/or survival of the CAL33 human HNSCC cell line in vitro. Cell number and viability was measured by the MTT assay after a 72h incubation with or without the compounds at the indicated concentrations.

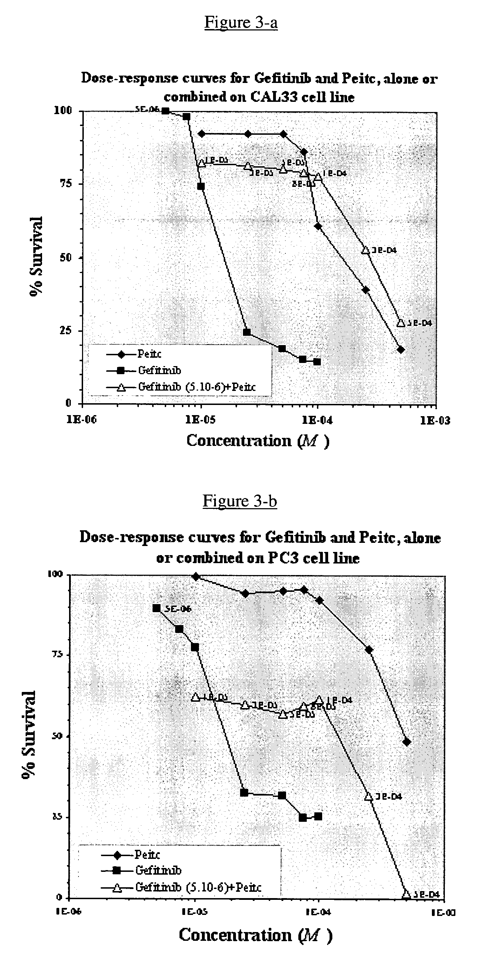

Figure 3-b: Effect of gefitinib and PEITC alone or combined on the growth and/or survival of the PC3 human HNSCC cell line in vitro. Cell number and viability was measured by the MTT assay after a 72h incubation with or without the compounds at the indicated concentrations.

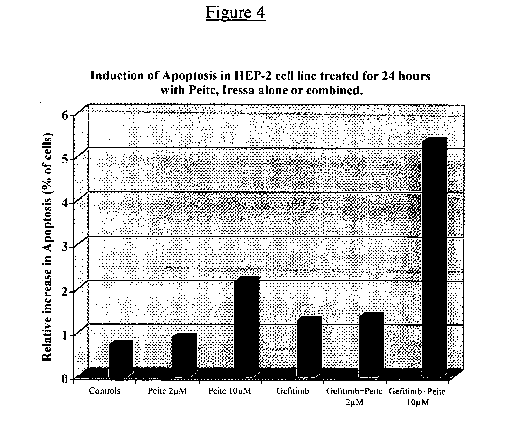

Figure 4: Rates of apoptosis as measured by the percentage of cells in sub-G1 by propidium iodide staining and FACS analysis of human HEP-2 HNSCC cells treated or not in vitro for 24h with the indicated compounds.

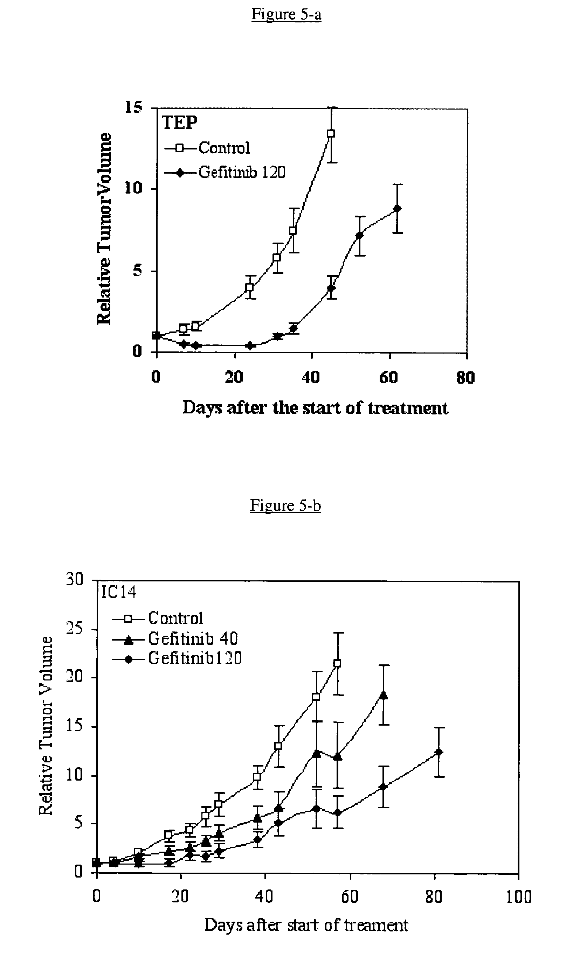

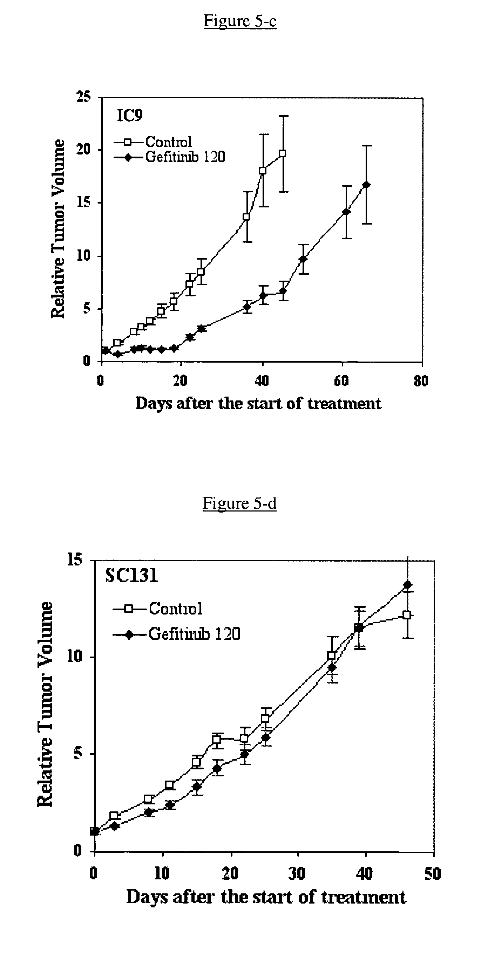

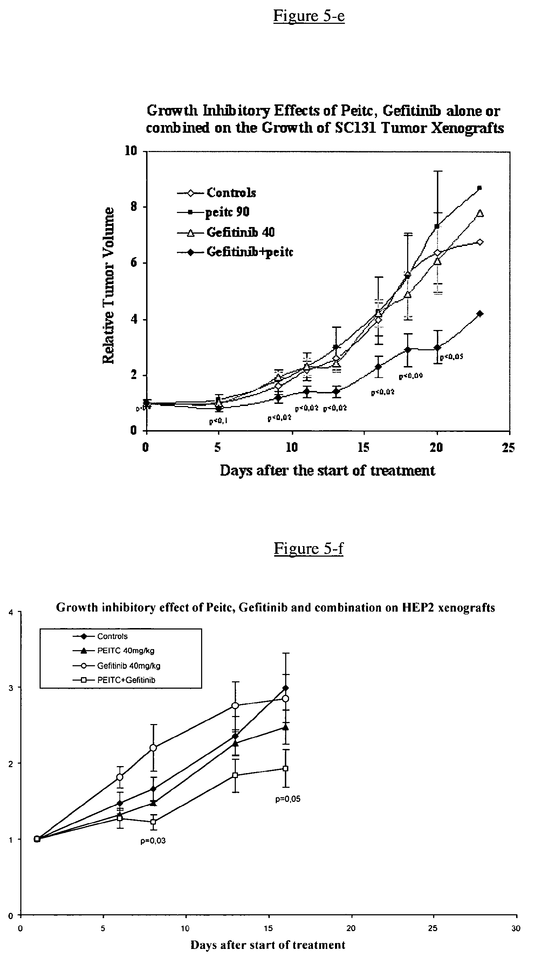

Figure 5a: Effect of Gefitinib administered at 120mg/kg on the growth of the human TEP NSCLC xenograft in nude mice.
Figure 5b: Effect of Gefitinib administered at 40 or 120mg/kg on the growth of the human IC14 NSCLC xenograft in nude mice.
Figure 5c: Effect of Gefitinib administered at 120mg/kg on the growth of the human IC9 NSCLC xenograft in nude mice.
Figure 5d: Effect of Gefitinib administered at 120mg/kg on the growth of the human SC131 NSCLC xenograft in nude mice.
Figure 5e: Effect of Gefitinib and PEITC alone or in combination on the growth of the human SC131 NSCLC xenograft in nude mice.
Figure 5f: Effect of Gefitinib and PEITC alone or in combination on the growth of the human HEP-2 HNSCC xenograft in nude mice.

### Gene expression analysis

*In vivo* studies were conducted with several human NSCLC xenografted in nude mice to assess the effect of gefitinib on tumor growth (see results in section 3 below). Two tumors differing by their sensitivity to gefitinib were selected for further studies: SC131 is refractory, and IC9 is sensitive to tumor growth inhibition by gefitinib.

Possible determinants of gefitinib sensitivity in these tumors were searched for by hybridizing radiolabeled tumor cDNA to macroarray membranes (Superarray, Tebu) spotted with 96 DNA sequences corresponding to genes involved in MAP kinase signaling.

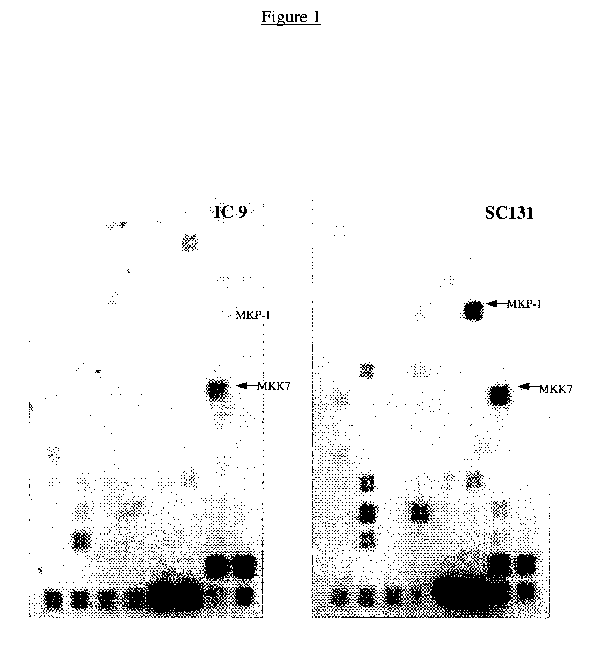

Among few other differentially expressed genes, MKP-1 was identified as a gene whose expression was high in the gefitinib-resistant SC131 tumor, and undetectable in the gefitinib-sensitive IC9 tumor (Fig.1)

MKP-1 codes for a DSP which inactivates JNK (see above). On the other hand, the MKK7/JNKK2 gene coding for a kinase that is a positive upstream regulator of JNK was expressed at similar levels in both tumors.

A real-time quantitative RT-PCR assay was then designed to study the expression levels of MKP-1 mRNA in a larger panel of tumors previously studied for their sensitivity to growth inhibition by gefitinib either *in vitro* or *in vivo* (see sections 2 and 3 below).

Since a high amount of MKP-1 relative to that of MKK7 is likely to be indicative of effective inhibition of JNK activity by MKP-1, the expression level of MKK7 mRNA was also measured, and the ratio of MKP-1 to MKK7 mRNA was used as a positive index of JNK signaling inhibition.

Oligonucleotide primers specific for the MKP-1 and MKK7 cDNA sequences were chosen based on sequence alignments of the known DSP and MKK7 family members cDNAs using the Clustal-X software (Genebank accession no. XM_037430.6, NM_001395.1, NM_004420.1, NM_001946.1, NM_004419.2, NM_001394.4, NM_004090.2, NM_020185.3, NM_022076.2, NM_004418.2, NM_080876.2, NM_152511.2, NM_080611.3, NM_152511.2, NM_080611.3, NM_007026.1, NM_016364.2, NM_007240.1, NM_003584.1, NM_007207.3, NM_004417.2, NM_005043.2, NM_145185.1, NM_145329.1). PCR primers were designed using the Primer-Express software (ABI, Les Ulys, France).

The sequences of the PCR primers used in this example are the following: MKP-1, forward: 5'-GACTTCATAGACTCCATCAAGAATGC, reverse: 5'-GGCAGTGGACAAACACCCTT; MKK7, forward: 5'-GGACCTGGATGTGGTGCTG, reverse: 5'-GAAGACGTCCGTGTTGGTGA.

Real-time PCR was performed with a SYBR-green-based assay (Eurogentec, Belgium) on a Gene-Amp 5700 Sequence Detector System (ABI), and relative mRNA levels were calculated by the comparative Ct method following validation by titration curve analysis.

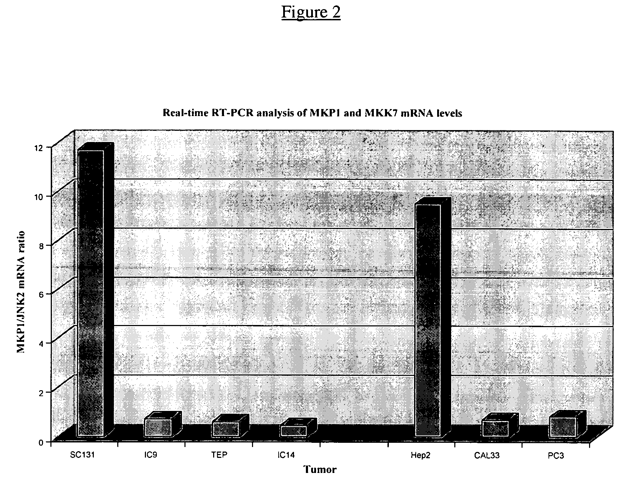

The data shown in Fig. 2 were obtained from four human NSCLC xenografts and three human HNSCC cell lines characterized for their response to gefitinib either *in vivo* or *in vitro* (see sections 2 and 3 below). The two gefitinib-resistant tumors (SC131 and Hep2) have a MKP-1/MKK7 ratio of 11 and 9 respectively, whereas the other gefitinib-sensitive tumors all have a MKP-1/MKK7 ratio below 1.

These data show that a high MKP-1/MKK7 mRNA ratio correlates with resistance to gefitinib treatment, suggesting that effective suppression of JNK activity by high constitutive MKP-1 levels protects tumor cells from growth inhibition and/or apoptosis induced by EGFR inhibition.

### In vitro studies of gefitinib sensitivity in three human HNSCC cell lines

The 3-(4,5-dimethylthiazol-2-yl)-2,5-diphenyltetrazolium bromide (MTT) cell proliferation assay was used to determine the growth inhibitory effect of gefitinib on the Hep2, CAL33 and PC3 human HNSCC cell lines. Cells were grown in 96-well plates and incubated with varying concentrations of gefitinib for 72h in DMEM medium supplemented with 10% foetal calf serum, 2 mM glutamine and antibiotics. Each measurement was done in quadruplicate and the data were expressed as % proliferation of control cells incubated in the absence of drug.

Phenylethyl isothiocyanate (PEITC) was used in addition to gefitinib, a known JNK inducer that acts through suppression of DUSPs activity, to look at the effect of MKP-1 inhibition on gefitinib sensitivity (11). The IC50 (i.e. drug concentration producing a 50% cell growth inhibition) of gefitinib was first determined and PEITC used alone, and then performed titration experiments with both drugs in combination with a fixed gefitinib concentration (5x10-6M).

The Hep2 cell line showed a 4-fold increase in the IC50 for gefitinib, compared with CAL33 and PC3, in agreement with previously published studies given in Table 1 hereinafter:

**Table 1**

| | | | |
|---|---|---|---|
| Tumor cell lines | CAL33 | PC3 | HEP-2 |
| IC₅₀ gefitinib™ | 1,8×10-5 | 1,9x10-5 | 7,5x10-5 |
| IC₅₀PEITC | 1,8×10-4 | 4,9x10-4 | 3,5x10-4 |
| Synergy | 0 | + | +++ |

In the combination study, a synergistic effect was obtained in the PC3 cell lines, while no additive effect was obtained in the CAL33 cell line (Fig. 3-a & 3-b).

Further studies concerned the effect of a 24h exposure to gefitinib (40 µM) and PEITC (2 or 10 µM) alone or in combination on the percentage of apoptotic cells in the gefitinib-resistant Hep2 cell line, as measured by FACS analysis of the % of cells in sub-G1 after propidium iodide staining (Fig. 4).

The data show that a strong synergistic anti-proliferative effect of the gefitinib/PEITC combination was obtained in cells with a high MKP-1/MKK7 mRNA ratio, and that a synergistic pro-apoptotic was obtained in gefitinib-resistant cells with a strong imbalance of the MKP-1/MKK7 ratio when gefitinib was combined with an inhibitor of DUSP activity.

### In vivo studies of human tumor xenografts

The effect of gefitinib and PEITC alone or in combination on the growth of human tumor xenografts in nude mice was tested according to previously published procedures.

Briefly, tumor xenografts were maintained by successive s.c. transplantation of tumor fragments in 8-week old nude mice. Tumor growth curves were obtained by plotting the mean relative tumor volume (i.e. the volume at a given time divided by the volume at the start of the experiment) from groups of at least 5 mice against time. Gefitinib was administered per os at 40 or 120 mg/kg 5 days a week for 3 to 5 weeks, while PEITC dissolved in corn oil was administered per os at 20 to 90 mg/kg every 2 days. No toxicity was observed in treated mice under experimental conditions, as judged by the absence of weight loss and clinical observation.

Three out of four NSCLC xenografts tested responded to gefitinib at the dose of 120 mg/kg and to a lesser extent at the dose of 40 mg/kg (Fig. 5-a, b & c). The SC131 NSCLC xenograft did not respond to gefitinib (Fig. 5-d).

In contrast, a significant inhibition of tumor growth was obtained with the combination of gefitinib (40 mg/kg) and

PEITC (20-90 mg/kg) in the SC131 NSCLC and the Hep2 HNSCC xenografts (Fig. 5-e & f). With these two tumors, neither gefitinib or PEITC had any antitumor effect when given alone.

These data show that DUSP inhibition by PEITC combined with EGFR inhibition by gefitinib has a synergistic antitumor activity in vivo in gefitinib-resistant tumors.

## Claims

1. Tumor markers predictive of the tumor cells resistance to inhibitors of RTK activity consisting of phosphatase/kinase expression ratio as measured in a tumor sample.

2. The tumor markers according to claim 1, consisting of MKP-1/MKK7 mRNA ratio.

3. A method for predicting tumor response to RTK inhibition, comprising measuring phosphatase/kinase mRNA level in tumors.

4. The method of claim 3, wherein said expression level is measured by using a real-time quantitative assay and calculating the relative mRNA levels by the comparative Ct method following validation by titration curve analysis.

5. The method according to claim 3 or 4, comprising the measurement of MKP-1/MKK7 mRNA ratio.

6. The method of claim 5, comprising the use of primers having sequences specific for the MKP-1 and MKK7 cDNA sequences, such as:
for MKP-1 Forward sequence SEQ ID N°1: 5′-GACTTCATAGACTCCATCAAGAATGC Reverse sequence SEQ ID N°2: 5'-GGCAGTGGACAAACACCCTT
For MKK7

Forward sequence SEQ ID N°3: 5′-GGACCTGGATGTGGTGCTG

Reverse sequence SEQ ID N°4: GAAGACGTCCGTGTTGGTGA

7. The use of the tumor markers according to claim 1 or 2, or a method according to any one of claims 3 to 6, comprising selecting patients with tumors susceptible to respond to RTK inhibitors, and further comprising administering to a patient an effective amount of a RTK inhibitor in combination with an inhibitor of phosphatase activity.

8. Pharmaceutical compositions comprising a RTK inhibitor together with an inhibitor of phosphatase activity.

9. Pharmaceutical compositions according to claim 8, wherein the RTK inhitor is gefitinib.

10. Pharmaceutical compositions according to claim 8 or 9, wherein the inhibitor of phosphatase is PEITC.


SEQUENCE LISTING

<110>  INSTITUT CURIE

      CENTRE NATIONAL DE LA RECHERCHE SCIENTIFIQUE (CNRS)

<120>  Means for detecting and treating cancer cells resistant to therapeutic
agents

<130>  CP/61224-1969

<140>  EP 04290378.1

<141>  2004-02-12

<160>  4

<170>  PatentIn version 3.1

<210>  1

<211>  26

<212>  DNA

<213>  Artificial

<400>  1
gacttcatag actccatcaa gaatgc                                          26

<210>  2

<211>  20

<212>  DNA

<213>  Artificial

<400>  2
ggcagtggac aaacaccctt                                                 20

<210>  3

<211>  19

<212>  DNA

<213>  Artificial

<400>  3
ggacctggat gtggtgctg                                                    19

<210>  4

<211>  20

<212>  DNA

<213>  Artificial

<400>  4
gaagacgtcc gtgttggtga                                                   20

### Annex to the application documents - subsequently filed sequences listing

SEQUENCE LISTING

<110> INSTITUT CURIE

CENTRE NATIONAL DE LA RECHERCHE SCIENTIFIQUE (CNRS)

<120> Means for detecting and treating cancer cells resistant to therapeutic
agents

<130> CP/61224-1969

<140> EP 04290378.1
<141> 2004-02-12

<160> 4

<170> PatentIn version 3.1

<210> 1
<211> 26
<212> DNA
<213> Artificial sequence

<220>
<223> Oligonucleotide primer
<400> 1
gacttcatag actccatcaa gaatgc 26

<210> 2
<211> 20
<212> DNA
<213> Artificial sequence

<220>
<223> Oligonucleotide primer
<400> 2
ggcagtggac aaacaccctt 20

<210> 3

<211> 19

<212> DNA

<213> Artificial sequence

<220>

<223> Oligonucleotide primer

<400> 3
ggacctggat gtggtgctg 19

<210> 4

<211> 20

<212> DNA

<213> Artificial sequence

<220>

<223> Oligonucleotide primer

<400> 4
gaagacgtcc gtgttggtga 20